# EUROPÄISCHE PATENTSCHRIFT

(11) **EP 0 730 430 B1**
(45) Veröffentlichungstag und Bekanntmachung des Hinweises auf die Patenterteilung: **28.01.1998**
(21) Anmeldenummer: 95900074.6
(22) Anmeldetag: 11.11.1994
(51) Int. Cl.: A61B 5/00, G01N 21/47

(54) **VORRICHTUNG ZUR UNTERSUCHUNG VON GEWEBE MIT LICHT**
DEVICE FOR EXAMINING TISSUES WITH LIGHT
DISPOSITIF POUR L'EXAMEN DE TISSUS A LA LUMIERE

(30) Priorität: 24.11.1993 DE 4340072
(43) Veröffentlichungstag der Anmeldung: 11.09.1996
(73) Patentinhaber: SIEMENS AKTIENGESELLSCHAFT, 80333 München (DE)
(72) Erfinder: KLINGENBECK-REGN, Klaus, D-90408 Nürnberg (DE)
(86) Internationale Anmeldenummer: DE9401332
(87) Internationale Veröffentlichungsnummer: WO9514427

(56) Entgegenhaltungen:
- DE-A- 2 950 780
- DE-A- 4 128 744
- DE-A- 4 327 798
- GB-A- 2 228 314

## Beschreibung

Die Erfindung betrifft eine Vorrichtung zur Untersuchung von Gewebe mit Licht. Derartige Vorrichtungen können mit sichtbarem, NIR- oder IR-Licht arbeiten. Die Wellenlänge des sichtbaren Lichtes liegt zwischen 380 und 780 nm, die von NIR-Licht, d.h. nahinfrarotem Licht, zwischen 780 nm und 1,5 µm und die von IR-Licht, also infrarotem Licht, zwischen 1,5 µm und 1 mm, wobei für Vorrichtungen der eingangs genannten Art der Wellenlängenbereich von 660 nm bis 1,2 µm besonders geeignet ist.

Viele optische Eigenschaften von Gewebe, z.B. die Absorption, die Streuung und die spektralen Eigenschaften, lassen sich durch Einstrahlung von Licht bestimmen. Es ist daher beispielsweise möglich, in der Mammadiagnostik Gewebeveränderungen festzustellen, indem Licht in die Mamma eingestrahlt, das aus dieser austretende Licht detektiert und die so gewonnene Information in geeigneter Weise ausgewertet wird.

Bei bekannten Vorrichtungen (siehe z.B. DE 41 28744 C1) tritt das Problem auf, daß sich die durch Detektion des austretenden Lichtes gewonnene Information nur unter Schwierigkeiten auswerten läßt, da der Kontrast zwischen etwaigen Inhomogenitäten und dem umgebenden Gewebe häufig zu gering ist.

Der Erfindung liegt daher die Aufgabe zugrunde, eine Vorrichtung der eingangs genannten Art so auszubilden, daß die durch Detektion des austretenden Lichtes gewonnene Information besser auswertbar ist.

Nach der Erfindung wird diese Aufgabe gelöst durch eine Vorrichtung zur Untersuchung von Gewebe mit Licht, aufweisend
a) Mittel zum bidirektionalen Durchstrahlen zu untersuchenden Gewebes mit Licht, und
b) Mittel zum Detektieren, die dazu vorgesehen sind, durch das zu untersuchende Gewebe transmittierte Anteile des von den Mitteln zum Detektieren ausgehenden Lichtes für beide Durchstrahlungsrichtungen zu detektieren.

Unter bidirektionaler Durchstrahlung soll hier verstanden werden, daß mit zwei entgegengesetzten Durchstrahlungsrichtungen gearbeitet wird, wobei die optische Achse für beide Durchstrahlungsrichtungen wenigstens im wesentlichen gleich ist. Bei der Durchstrahlung von Gewebe heben sich infolge der starken Streuung, der Licht in Gewebe unterliegt, Inhomogenitäten bzw. Absorber um so kontrastreicher von dem umgebenden Gewebe ab, je näher sie sich bei den Mitteln zum Detektieren, also derjenigen Oberfläche befinden, durch die das zu detektierende Licht aus dem Gewebe austritt. Da im Falle der erfindungsgemäßen Vorrichtung bidirektional durchstrahlt wird, ergibt sich außer für den Sonderfall, daß eine Inhomogenität für beide Durchstrahlungsrichtungen den gleichen Abstand zu den Mitteln zum Detektieren aufweist, für die beiden Durchstrahlungsrichtungen ein unterschiedlicher Kontrast, mit dem sich die Inhomogenität von dem umgebenden Gewebe abhebt. Es steht also besser auswertbare Information zur Verfügung, da jeweils die günstigere, d.h. kontrastreichere Information herangezogen und der Auswertung zugrundegelegt werden kann. Zugleich wird eine Information darüber gewonnen, ob sich eine Inhomogenität dichter bei der einen oder der anderen den durchstrahlten Gewebebereich in Durchstrahlungsrichtung begrenzenden Fläche liegt.

Um zu verhindern, daß sich die Eigenschaften des durchstrahlten Gewebes wahrend der zwischen der Durchstrahlung in der einen und der anderen Durchstrahlungsrichtung verstreichenden Zeit signifikant ändern können, sieht eine Variante der Erfindung vor, daß die bidirektionale Durchstrahlung wenigstens im wesentlichen quasi-gleichzeitig erfolgt. In diesem Zusammenhang ist gemäß einer Variante der Erfindung vorgesehen, daß die Mittel zum Durchstrahlen das zu untersuchende Gewebe in beiden Durchstrahlungsrichtungen tatsachlich gleichzeitig durchstrahlen. Dabei kann eine gegenseitige Beeinflussung der beiden Durchstrahlungsrichtungen dadurch vermieden werden, daß das Licht für die beiden Durchstrahlungsrichtungen jeweils unterschiedlich moduliert ist. Vorzugsweise ist das Licht für die beiden Durchstrahlungsrichtungen mit unterschiedlichen Modulationsfrequenzen amplitudenmoduliert. Infolge der unterschiedlichen Modulation ist es leicht möglich, für die beiden Durchstrahlungsrichtungen jeweils nur das zugehörige Licht zu berücksichtigen, indem das Ausgangssignal der Mittel zum Detektieren in geeigneter Weise demoduliert wird.

Um gegenseitige Beeinflussungen der beiden Durchstrahlungsrichtungen zu verhindern, können auch Blendenmittel vorgesehen sein, die alternierend für die eine oder die andere Durchstrahlungsrichtung das von den Mitteln zum Durchstrahlen ausgehende Licht von den Mitteln zum Detektieren fernhalten.

Gemäß einer Variante der Erfindung weisen die Mittel zum Durchstrahlen eine erste und eine zweite Lichtquelle und die Mittel zum Detektieren eine erste und eine zweite Detektoreinrichtung auf, wobei von der ersten Lichtquelle das Licht in der einen Durchstrahlungsrichtung ausgeht und die erste Detektoreinrichtung zur Detektion durch das Gewebe transmittierter Anteile des von der ersten Lichtquelle ausgehenden Lichtes vorgesehen ist, und wobei von der zweiten Lichtquelle das Licht in der anderen Durchstrahlungsrichtung ausgeht und die zweite Detektoreinrichtung zur Detektion durch das Gewebe transmittierter Anteile des von der zweiten Lichtquelle ausgehenden Lichtes vorgesehen ist. Gemäß einer Ausführungsform der Erfindung kann dann vorgesehen sein, daß die erste Lichtquelle und die erste Detektoreinrichtung einerseits und die zweite Lichtquelle und die zweite Detektoreinheit andererseits gleichzeitig aktiv sind. Es kann aber auch vorgesehen sein, daß die erste Lichtquelle und die erste Detektoreinrichtung einerseits oder die zweite Lichtquelle und die zweite Detektoreinrichtung andererseits alternierend aktiv sind.

Weiter kann gemäß einer Variante der Erfindung vorgesehen sein, daß die Mittel zum Durchstrahlen eine Lichtquelle aufweisen, der Lichtleitmittel zugeordnet sind, die das Licht der Lichtquelle alternierend in der einen oder der anderen Durchstrahlungsrichtung aussenden. Auf diese Weise läßt sich der zu treibende technische Aufwand verringern. Dies gilt auch dann, wenn die Mittel zum Detektieren eine Detektoreinrichtung aufweisen, der Lichtleitmittel zugeordnet sind, die dazu vorgesehen sind, der Detektoreinrichtung alternierend bezüglich der eine oder der anderen Durchstrahlungsrichtung zu detektierendes Licht zuzuleiten.

Die Lichtleitmittel können beispielsweise Strahlteiler und Spiegel enthalten. Gemäß einer wegen ihrer technischen Einfachheit bevorzugten Ausführung der Erfindung enthalten die Lichtleitmittel faseroptische Mittel, z.B. Lichtleitfasern. Die faseroptischen Mittel können auch ein Lichtleitfaserbündel enthalten, das wenigstens je eine von den Mitteln zum Durchstrahlen ausgehendes Licht einer Durchstrahlungsrichtung zu dem zu untersuchenden Gewebe und eine transmittierte Anteile Lichtes der jeweils anderen Durchstrahlungsrichtung zu den Mitteln zum Detektieren leitende Lichtleitfaser enthält. Insbesondere für den Fall, daß beiderseits des zu untersuchenden Gewebes derartige Lichtleitfaserbündel verwendet werden, ergibt sich ein besonders hohes Maß an Übereinstimmung der optischen Achsen für die beiden Durchstrahlungsrichtungen.

Um auch größere Gewebebereiche untersuchen zu können, sieht eine besonders bevorzugte Variante der Erfindung vor, daß Abtastmittel vorgesehen sind, mittels derer die bidirektionale Durchstrahlung des zu untersuchenden Gewebes in einer Vielzahl von Abtastpositionen erfolgt. Es besteht dann also die Möglichkeit, im Transmissionsverfahren sozusagen Bilder des zu untersuchenden Gewebebereiches zu erstellen. Dies geschieht mit Hilfe von Auswertemitteln, denen die Ausgangssignale der Mittel zum Detektieren zugeführt sind. Für den Fall, daß infolge abweichender optischer Achsen ein Versatz der beiden Durchstrahlungsrichtungen vorliegt, kann gemäß einer Variante der Erfindung vorgesehen sein, daß die Auswertemittel den Versatz eliminieren. Dies kann durch in der Bildverarbeitung gängige Verfahren geschehen. Besonders leicht läßt sich ein Versatz eliminieren, wenn der Versatz nach Betrag und Richtung gleich dem Versatz zwischen zwei benachbarten Abtastpositionen oder einem ganzzahligen Vielfachen davon ist. Der Versatz zwischen den für die beiden Durchstrahlungsrichtungen erstellten Bildern läßt sich dann eliminieren, indem zu zueinander gehörigen Abtastpositionen gehörige Bildpunkte einander zugeordnet werden.

Um auch spektroskopische Untersuchungen durchführen zu können, sieht eine Variante der Erfindung vor, daß die Mittel zum Durchstrahlen gleichzeitig Licht definierter unterschiedlicher Wellenlängen abgeben, wobei die Mittel zum Detektieren eine der Anzahl der unterschiedlichen Wellenlängen entsprechende Anzahl von Signalen abgeben, von denen jedes dem Anteil des Lichtes einer der unterschiedlichen Wellenlängen an dem detektierten Anteil des von den Mitteln zum Durchstrahlen ausgehenden durch das Gewebe transmittierten Lichtes entspricht.

Ausführungsbeispiele der Erfindung sind in den beigefügten Zeichnungen dargestellt. Es zeigen:
- Fig. 1 bis 3: in grob schematischer, teilweise blockschaltbildartiger Darstellung erfindungsgemäße Vorrichtungen,
- Fig. 4: eine weitere erfindungsgemäße Vorrichtung in grob schematischer, blockschaltbildartiger Darstellung,
- Fig. 5: ein Detail der Vorrichtung gemäß Fig. 4, und
- Fig. 6: eine Variante der Vorrichtung gemäß Fig. 4 und 5.

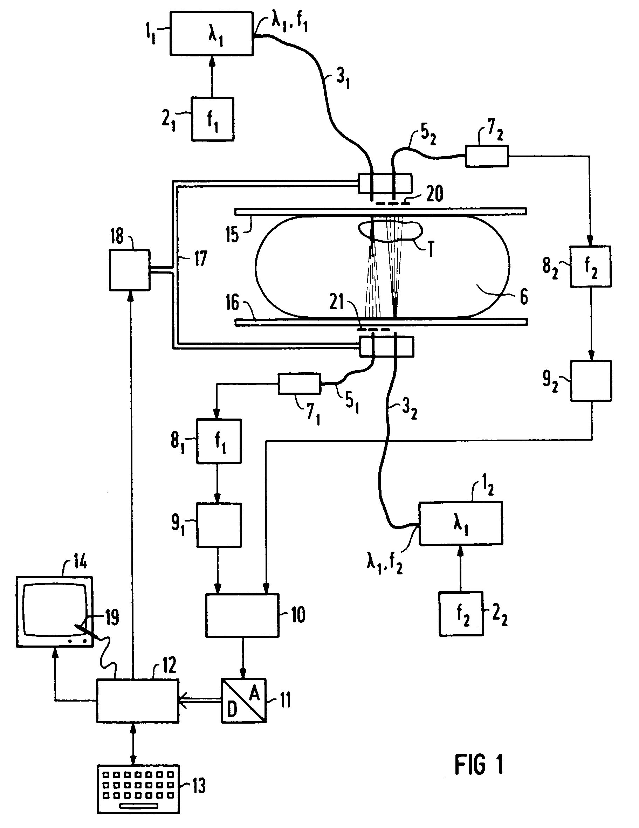

In Fig. 1 ist eine erfindungsgemäße Vorrichtung dargestellt, die beispielsweise zur Mammadiagnostik verwendet werden kann. Die Vorrichtung weist zwei Lichtquellen 1₁ und 1₂ auf, von denen jede kohärentes Licht der Wellenlänge λ₁ abgibt. Jede der Lichtquellen 1₁ und 1₂ enthält eine Halbleiter-Laserdiode und die jeweils zugehörige Stromversorgung. Beiden Lichtquellen 1₁ und 1₂ ist ein elektrischer Signalgenerator 2₁ und 2₂ zugeordnet, der der in der jeweiligen Lichtquelle 1₁ bzw. 1₂ enthaltenen Stromversorgung ein Wechselstromsignal fester Frequenz zuführt, mittels dessen der Versorgungsstrom der in der jeweiligen Lichtquelle 1₁ bzw. 1₂ enthaltenen Halbleiter-Laserdiode moduliert wird. Jeder der Signalgeneratoren 2₁ und 2₂ erzeugt ein Wechselstromsignal einer anderen Frequenz f₁ bzw. f₂. Da die Amplitude bzw. die Intensität des von den Laserdioden abgegebenen Lichtes der Stromstärke ihres Versorgungsstromes im wesentlichen proportional ist, geben die Lichtquellen 1₁ und 1₂ also Licht der Wellenlänge λ₁ ab, das mit einer jeweils anderen Modulationsfrequenz f₁ bzw. f₂ amplitudenmoduliert ist.

Das von den Lichtquellen 1₁ und 1₂ abgegebene Licht wird über faseroptische Lichtwellenleiter 3₁ bzw. 3₂ dem zu untersuchenden Gewebe, d.h. dem Objekt 6, beispielsweise einer Mamma, zugeführt. Das Objekt 6 ist zwischen zwei planen, parallel zueinander angeordneten, für das Licht der Lichtquellen 1₁ und 1₂ im wesentlichen transparenten Kompressionsplatten 15 und 16 angeordnet.

Die Enden der Lichtwellenleiter 3₁ und 3₂, die die Lichtaustrittszonen der Vorrichtung bilden, sind einander annähernd gegenüberliegend auf unterschiedlichen Seiten des Objektes 6 angeordnet, und zwar derart, daß, wenn man von der optischen Streuung in dem Gewebe des Objektes 6 absieht, die von dem Licht der beiden Lichtquellen 1₁ und 1₂ - in entgegengesetzter Richtung - durchlaufenen Gewebebereiche, anders als dies aus Gründen der Übersichtlichkeit in Fig. 1 dargestellt ist, sich nicht wesentlich voneinander unterscheiden. Ein zu untersuchender Gewebebereich des Objektes 6 kann also mit von den Lichtquellen 1₁ und 1₂ stammendem Licht bidirektional durchstrahlt werden.

Zur Detektion der durch das Objekt 6 transmittierten Anteile des eingestrahlten Lichtes sind zwei Photomultiplier 7₁ und 7₂ vorgesehen, denen die aus dem Objekt 6 austretenden Anteile des transmittierten Lichtes über faseroptische Lichtwellenleiter 5₁ bzw. 5₂ zugeführt ist. Die freien Enden der Lichtwellenleiter 51 und 5₂, die die Lichteintrittszonen der Vorrichtung bilden, sind ebenso wie die freien Enden der Lichtleiter 3₁ und 3₂ möglichst dicht bei dem zu untersuchenden Objekt 6, d.h. bei der entsprechenden Kompressionsplatte 15 bzw. 16, angeordnet.

Es versteht sich, daß mittels des Photomultipliers 7₁ der durch das Objekt 6 transmittierte Anteil des von der Lichtquelle 1₁ stammenden Lichtes und mittels des Photomultipliers 7₂ der durch das Objekt in entgegengesetzter Richtung transmittierte Anteil des von der Lichtquelle 1₂ stammenden Lichtes detektiert werden soll. Die Ausgangssignale der Photomultiplier 7₁ und 7₂ sind einem Bandpaßfilter 8₁ bzw. 8₂ zugeführt. Die Mittenfrequenzen der Bandpaßfilter 8₁ und 8₂ sind derart gewählt, daß sie möglichst exakt mit den Frequenzen f₁ bzw. f₂ übereinstimmen, mit denen das Licht der Lichtquellen 1₁ bzw. 1₂ amplitudenmoduliert ist. Am Ausgang der Bandpaßfilter 8₁ und 8₂ stehen also Signale zur Verfügung, die ausschließlich die durch das Objekt 6 transmittierten Anteile des Lichtes der Lichtquelle 1₁ bzw. der Lichtquelle 1₂ repräsentieren. Diejenigen Anteile der Ausgangssignale der Photomultiplier 7₁ und 7₂, die das Umgebungslicht und von der Lichtquelle 1₂ bzw. 1₁ stammendes Licht repräsentieren, können die Bandpaßfilter 8₁ bzw. 8₂ nicht passieren.

Den Bandpaßfiltern 8₁ und 8₂ sind Signalverarbeitungsmittel in Form je einer Signalaufbereitungsschaltung 9₁ bzw. 9₂ nachgeschaltet, die eine dem jeweiligen Untersuchungsfall angepaßte Signalaufbereitung, beispielsweise durch Gleichrichtung, Glättung oder Integration, bewirken. Die Ausgangssignale der Signalaufbereitungsschaltungen 9₁ und 9₂ sind einem 2:1-Analog-Multiplexer 10 zugeführt, dessen Ausgang mit dem Eingang eines Analog/Digital-Wandlers 11 verbunden ist. Die digitalen Ausgangsdaten des Analog/Digital-Wandlers 11 gelangen zu einer elektronischen Recheneinrichtung 12, die u.a. der Steuerung der Vorrichtung dient und an die eine der Bedienung der Meßvorrichtung dienende Tastatur 13 und ein Monitor 14 angeschlossen sind.

Um Daten bezüglich größerer Bereiche des Objektes 6 sammeln zu können, sind die Lichtwellenleiter 3₁ und 5₂ einerseits und 3₂ und 5₁ andererseits an einem Träger 17 angebracht, der mittels einer Verstelleinheit 18, die von der elektronischen Recheneinrichtung 12 gesteuert wird, derart verstellt werden kann, daß die Lichtaustritts- und die Lichteintrittszonen der Meßvorrichtung nach Art einer Abtastbewegung gemeinsam relativ zu dem Objekt 6 verstellt werden können. Beispielsweise kann vorgesehen sein, daß im Zuge der Abtastbewegung Daten für 100 Stellungen des Trägers 17 gesammelt werden, welche Stellungen matrixartig in jeweils zehn Zeilen und Spalten angeordnet sind und sowohl in Zeilen- als auch in Spaltenrichtung jeweils den gleichen Abstand voneinander aufweisen. Die bei der vorzugsweise mäanderartigen Abtastbewegung erhaltenen Daten stellt die elektronische Recheneinrichtung 12 auf dem Monitor 14 vorzugsweise graphisch dar, wobei unterschiedliche Intensitäten des detektierten Lichtes durch unterschiedliche Grau- oder Farbwerte veranschaulicht werden.

Da bei der Durchstrahlung von Gewebe mit Licht eine Inhomogenität um so kontrastreicher dargestellt wird, je näher sie sich bei derjenigen Begrenzungsfläche des Objektes befindet, der der Detektor bzw. die Lichteintrittszone zugeordnet ist, und im Falle der erfindungsgemäßen Vorrichtung das Objekt 6 bidirektional durchstrahlt wird, werden bei der Abtastung des Objektes 6 sozusagen zwei bezüglich der Darstellung einer Inhomogenität, beispielsweise eines Tumors T, unterschiedlich kontrastreiche "Bilder" angefertigt, von denen das eine der einen und das andere der anderen Durchstrahlungsrichtung zugeordnet ist. Die zu den beiden Bildern gehörigen Daten speichert die elektronische Recheneinrichtung 12 in einer solcher Weise, daß sie jederzeit in der Lage ist zu erkennen, welche Daten zu welchem der beiden Bilder gehören.

In einer ersten Betriebsweise der Vorrichtung stellt die elektronische Recheneinrichtung 12 gleichzeitig zwei Bilder auf dem Monitor 14 dar, die den beiden Durchstrahlungsrichtungen entsprechen. Mittels eines Lichtgriffels 19 kann eine Bedienperson nun dasjenige Bild auswählen, auf dem die jeweils interessierende Inhomogenität kontrastreicher dargestellt ist. Das entsprechende Bild wird dann allein in vergrößertem Format auf dem Monitor 14 angezeigt.

In einer zweiten Betriebsweise besteht die Möglichkeit, mittels des Lichtgriffels 19 in einem der beiden Bilder einen oder mehrere Bereiche zu markieren, worauf die elektronische Recheneinrichtung 12 aus dem anderen Bild die entsprechenden Bereiche aus- und statt dessen die markierte Bereiche einblendet. Diese Betriebsweise ist insbesondere dann von Vorteil, wenn mehrere Inhomogenitäten vorhanden sind, die teils in dem einen und teils in dem anderen Bild kontrastreicher dargestellt sind, da dann eine oder mehrere in dem einen Bild weniger kontrastreich dargestellten Inhomogenitäten durch die entsprechenden Ausschnitte des anderen Bildes ersetzt werden kann bzw. können.

In einer weiteren Betriebsweise der Vorrichtung wertet die elektronische Recheneinrichtung 12 mit an sich bekannten Methoden der Bildverarbeitung die beiden unterschiedlichen Durchstrahlungsrichtungen entsprechenden Bilder aus und bildet aus den Daten beider Bilder ein einziges, maximal kontrastreiches Bild, das auf dem Monitor 14 angezeigt wird.

Aus den vorstehenden Ausführungen wird deutlich, daß die bidirektionale Durchstrahlung des Objektes 6 gleichzeitig erfolgt, was selbstverständlich von Vorteil ist, da für die einzelnen Abtastpositionen die den beiden Durchstrahlungsrichtungen entsprechenden Daten gleichzeitig und damit unter identischen Bedingungen ermittelt werden. Unter Umständen kann aber an der Oberfläche des Objektes 6 reflektiertes Licht der Lichtquelle 1₁ bzw. der Lichtquelle 1₂ zu Überbelichtungen des Photomultipliers 7₂ bzw. 7₁ führen, mit der Folge, daß Meßfehler auftreten und unter Umständen sogar einer der beiden bzw. beide Photomultiplier 7₁, 7₂ beschädigt werden. Um hier Abhilfe zu schaffen, kann einerseits vorgesehen sein, daß die elektronische Recheneinrichtung 12 die Lichtquellen 1₁ und 1₂ in nicht näher dargestellter Weise alternierend aktiviert. Alternativ besteht die Möglichkeit, an dem Träger 17 angebrachte, in Fig. 1 schematisch angedeutete Blendenmittel 20 bzw. 21 vorzusehen, die ähnlich einem Kameraverschluß aufgebaut sein können und alternierend derart bestätigt werden, daß das Licht der Lichtquelle 1₁ von dem Photomultiplier 7₂ bzw. das Licht der Lichtquelle 1₂ von dem Photomultiplier 7₁ ferngehalten wird. Auch die alternierende Betätigung der Blendenmittel 20 bzw. erfolgt in nicht dargestellter Weise durch die elektronische Recheneinrichtung 12. Im Falle der Fig. 1 sind die Blendenmittel 20 und 21 zwischen dem Objekt 6 und dem freien Ende der Lichtleitfaser 5₁ bzw. 5₂ angeordnet. Selbstverständlich besteht auch die Möglichkeit, die Blendenmittel 20 bzw. 21 zwischen dem Objekt 6 und den freien Enden der Lichtleitfasern 3₂ und 3₁ anzuordnen. Weiter besteht die Möglichkeit, ein Blendenmittel dem freien Ende einer der Lichtleitfasern 3₁ oder 3₂ zuzuordnen und das andere Blendenmittel dem freien Ende eines des zu der jeweils anderen Durchstrahlungsrichtung gehörigen Lichtwellenleiters 5₁ bzw. 5₂ zuzuordnen.

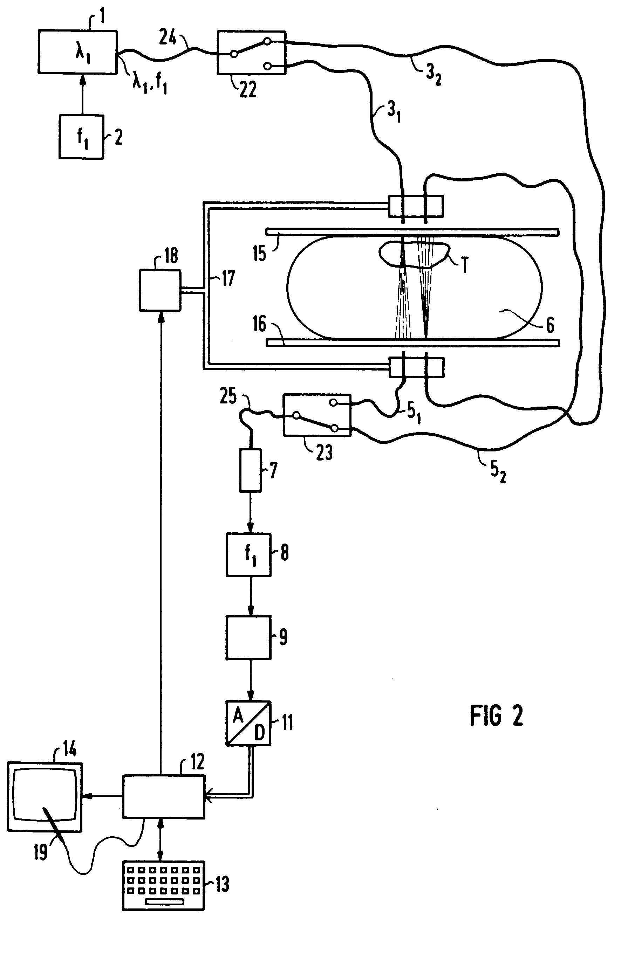

Die Vorrichtung gemäß Fig. 2 unterscheidet sich von der zuvor beschriebenen zunächst dadurch, daß nur eine Lichtquelle 1 mit Signalgenerator 2 vorgesehen ist, die mit der Frequenz f₁ amplitudenmoduliertes Licht der Wellenlänge λ₁ erzeugt, daß nur ein Photomultiplier 7, ein Bandpaßfilter 8 mit der Mittenfrequenz f₁ und eine Signalaufbereitungsschaltung 9 vorhanden sind und daß der Multiplexer 10 fehlt.

Um das Objekt 6 dennoch bidirektional durchstrahlen zu können, sind zwei Umschalteinrichtungen 22, 23 vorgesehen, bei denen es sich um optomechanische oder elektrooptische Schalter handeln kann. Der Umschalter 22, mit dem die Lichtquelle 1 über einen vorzugsweise faseroptischen Lichtwellenleiter 24 verbunden ist, dient dazu, das Licht der Lichtquelle 1 wahlweise in den Lichtwellenleiter 3₁ oder den Lichtwellenleiter 3₂ einzukoppeln und somit von der einen oder anderen Seite des Objektes 6 her in dieses einzustrahlen. Der Umschalter 23, der mit dem Photomultiplier 7 über einen vorzugsweise faseroptischen Lichtwellenleiter 25 verbunden ist, dient dazu, den Photomultiplier 7 wahlweise mit dem Lichtwellenleiter 5₁ oder dem Lichtwellenleiter 5₂ zu verbinden. Die Umschalter werden in nicht dargestellter Weise von der elektronischen Recheneinrichtung 12 derart alternierend betätigt, daß entweder über die Lichtleitfaser 3₁ das Licht der Lichtquelle 1 in das Objekt 6 eingestrahlt und die mittels des Lichtwellenleiters 5₁ empfangenen, durch das Objekt 6 transmittierten Anteile des Lichtes dem Photomultiplier 7 zugeführt werden oder das Licht der Lichtquelle 1 über den Lichtwellenleiter 3₂ dem Objekt 6 zugeführt wird und die mittels des Lichtwellenleiters 5₂ empfangenen, durch das Objekt 6 transmittierten Anteile dem Photomultiplier 7 zugeführt werden. Die Umschaltung erfolgt mit einer Frequenz von beispielsweise 200 Hz. Es wird also deutlich, daß das Objekt 6 quasi-gleichzeitig bidirektional durchstrahlt wird.

Die Vorrichtung gemäß Fig. 2 bietet den Vorteil eines verringerten Aufwandes, da der für die beiden Umschalter 22 und 23 zu treibende Aufwand geringer ist, als die Einsparungen, die dadurch erreicht werden, daß nur eine Lichtquelle 1, ein Photomultiplier 7, ein Bandpaßfilter 8, eine Signalaufbereitungsschaltung 9 und kein Multiplexer benötigt werden.

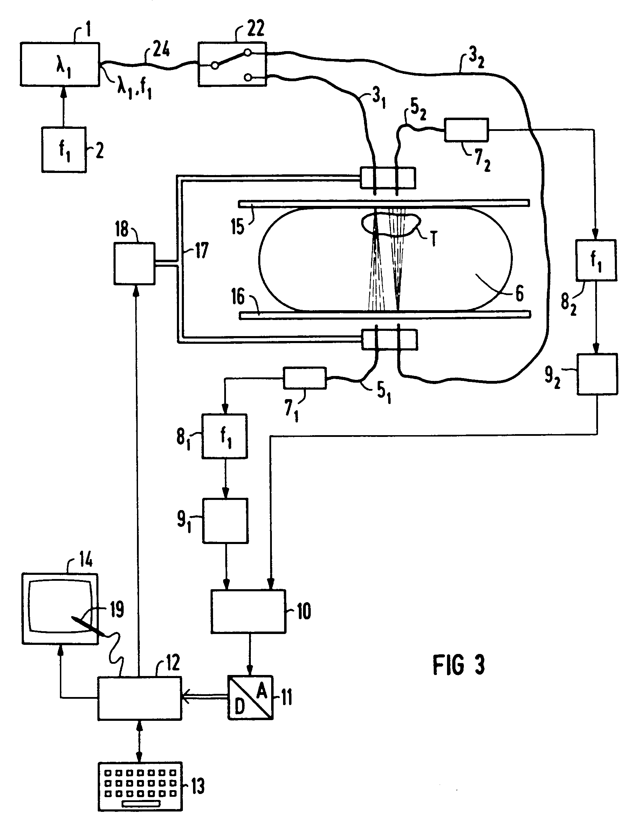

Wie anhand der Ausführungsform gemäß Fig. 3 beispielhaft deutlich wird, besteht auch die Möglichkeit, von Mischformen zwischen den Ausführungsformen gemäß den Fig. 1 und 2. So ist die Ausführungsform gemäß Fig. 3 "senderseitig" analog zur Fig. 2 aufgebaut, d.h., es ist nur eine Lichtquelle 1 vorhanden, deren Licht mittels des Umschalters 22 wahlweise in den Lichtwellenleiter 3₁ oder den Lichtwellenleiter 3₂ eingekoppelt werden kann. "Empfangsseitig" ist die Ausführungsform gemäß Fig. 3 analog zu der gemäß Fig. 1 ausgebildet, d.h. es sind zwei Photomultiplier 7₁ und 7₂, zwei Bandpaßfilter 8₁ und 8₂, zwei Signalaufbereitungsschaltungen 9₁ und 9₂ und ein 2:1-Analog-Multiplexer 10 vorgesehen. Da nur eine Lichtquelle 1 verwendet wird, deren Licht mit der Frequenz f₁ amplitudenmoduliert ist, haben beide Bandpaßfilter 8₁ und 8₂ die Mittenfrequenz f₁.

Es kann auch eine Vorrichtung realisiert werden, die "senderseitig" gemäß Fig. 1 und "empfängerseitig" gemäß Fig. 2 ausgebildet ist.

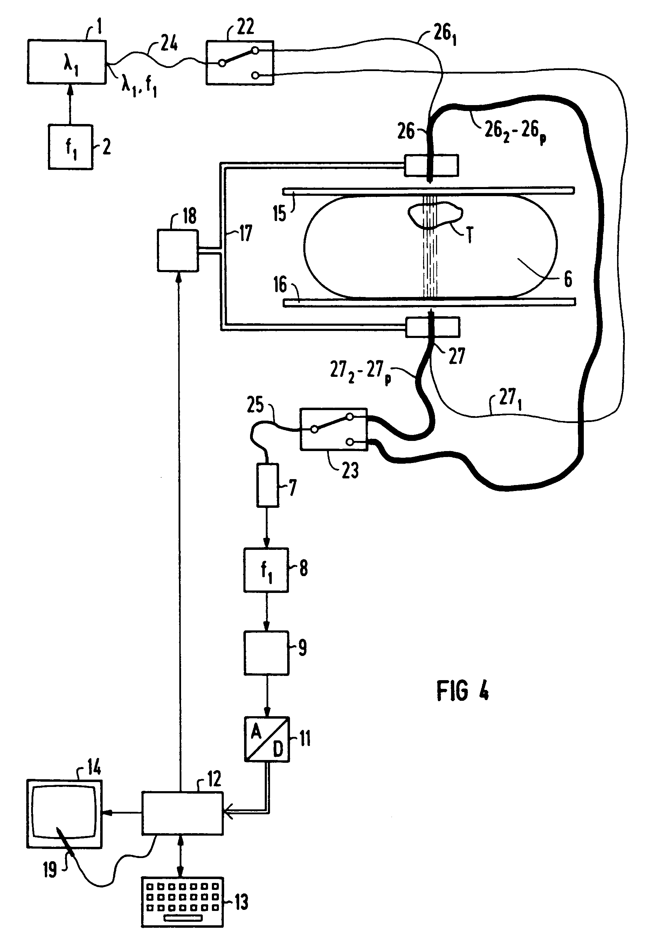

Die Ausführungsform gemäß Fig. 4 unterscheidet sich von der gemäß Fig. 2 dadurch, daß anstelle der Lichtwellenleiter 3₁ und 3₂ sowie 5₁ und 5₂ zwei Lichtleitfaserbündel 26 und 27 vorgesehen sind, die jeweils aus einer Anzahl von p Lichtleitfasern zusammengesetzt sind.

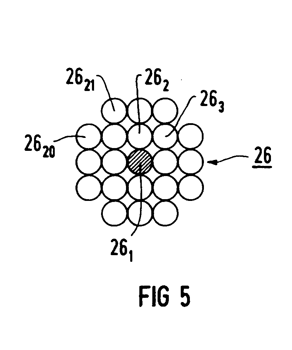

Die zentrale Lichtleitfaser 26₁ des Lichtleitfaserbündels 26 und die zentrale Lichtleitfaser 27₁ des Lichtleitfaserbündels 27 sind mit dem Umschalter 22 verbunden und entsprechen in ihrer Funktion den Lichtwellenleitern 3₁ und 3₂ des Ausführungsbeispieles gemäß Fig. 2. Die verbleibenden Lichtleitfasern 26₂ bis 26ₚ bzw. 27₂ bis 27ₚ der Lichtleitfaserbündel 26 und 27 sind mit dem Umschalter 23 verbunden und entsprechen in ihrer Funktion den Lichtwellenleitern 5₁ und 5₂ des Ausführungsbeispieles gemäß Fig. 2. Das freie Ende des Lichtleitfaserbündels 26 ist übrigens in Fig. 5 vergrößert dargestellt, wobei 21 Lichtleitfasern 26₁ bis 26₂₁ vorgesehen sind (p=21), von denen in der Fig. 5 nur einige mit Bezugszeichen versehen sind, und die zentrale Lichtleitfaser 26₁ schraffiert dargestellt ist.

Die freien Enden der Lichtleitfaserbündel 26 und 27 fungieren also je nach Durchstrahlungsrichtung als Lichtaustritts- oder Lichteintrittszone. Die freien Enden der Lichtleitfaserbündel 26 und 27 sind in dem Träger 17 derart aufgenommen, daß bei Abwesenheit eines Objektes 6 das aus der zentralen Lichtleitfaser 26₁ bzw. 27₁ austretende Licht in die jeweils andere zentrale Lichtleitfaser 27₁ bzw. 26₁ eintritt. Dadurch ist im Gegensatz zu den zuvor beschriebenen Ausführungsbeispielen, wo geringfügige Abweichungen des in den beiden Durchstrahlungsrichtungen von dem Licht durchlaufenen Gewebes vorliegen, eine exakt bidirektionale Durchstrahlung möglich.

Infolge des Umstandes, daß eine Vielzahl von Lichtleitfasern 26₂ bis 26ₚ bzw. 27₂ bis 27ₚ dazu dient, aus dem Objekt 6 austretende Anteile des transmittierten Lichtes aufzunehmen, ergibt sich ein verbesserter Signal-Störabstand. Der transmittierte Anteil des Lichtes tritt nämlich infolge von Streuungserscheinungen nicht an einer der Einstrahlstellen gegenüberliegenden und dieser in ihrer Größe entsprechenden Stelle, sondern in einem letztere Stelle umgebenden größeren Bereich aus dem Objekt 6 aus.

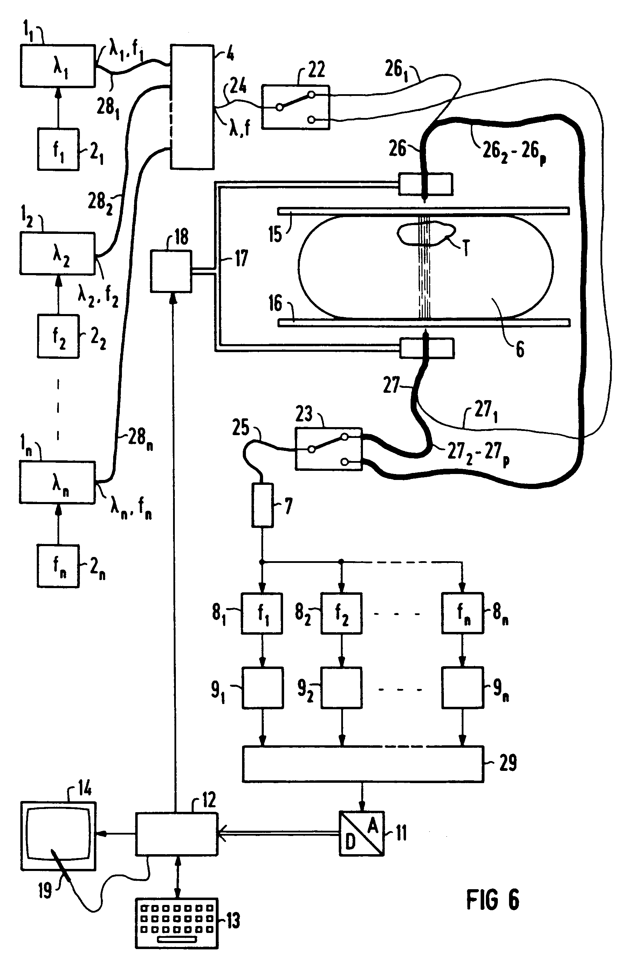

Die Ausführungsform gemäß Fig. 6 unterscheidet sich von der gemäß den Fig. 4 und 5 nochmals dadurch, daß auch spektroskopische Untersuchungen möglich sind. Hierzu ist eine Anzahl von Lichtquellen 1₁ bis 1ₙ vorgesehen, die Licht unterschiedlicher Wellenlängen λ₁ bis λₙ erzeugen, das mit Hilfe der Signalgeneratoren 2₁ bis 2ₙ mit unterschiedlichen Frequenzen f₁ bis fₙ amplitudenmoduliert ist. Das Licht der Lichtquellen 1₁ bis 1ₙ wird über insbesondere faseroptische Lichtwellenleiter 28₁ bis 28ₙ einem Lichtwellenleiter-Fan-In-Koppler 4 zugeführt, der n-Eingänge, mit denen jeweils einer der Lichtwellenleiter 28₁ bis 28ₙ verbunden ist, und einen Ausgang aufweist, an den der Lichtwellenleiter 24 angeschlossen ist.

Im Falle des Ausführungsbeispieles gemäß Fig. 6 wird also in das Objekt 6 Licht eingestrahlt, das sich durch die Überlagerung des von den Lichtquellen 1₁ bis 1ₙ jeweils abgegebenen Lichtes mittels des Lichtwellenleiter-Fan-In-Kopplers 4 ergibt. Dem Objekt 6 wird also Licht der unterschiedlichen Wellenlängen λ₁ bis λₙ für die jeweilige Durchstrahlungsrichtung an einem für alle Wellenlängen λ₁ bis λₙ jeweils gleichen Ort gleichzeitig zugeführt.

Um aus dem dem empfangenen Anteil des durch das Objekt 6 transmittierten Lichtes entsprechenden Signal des Photomultiplier 7, dessen zeitlicher Verlauf den zeitlichen Verlauf der Intensität des empfangenen Lichtes insoweit repräsentiert, als er der Amplituden-Hüllkurve des empfangenen Lichtes entspricht, Signale bezüglich der Intensitäten des Lichtes der unterschiedlichen Wellenlängen λ₁ bis λₙ gewinnen zu können, sind Demodulatormittel in Form von Bandpaßfiltern 8₁ bis 8ₙ vorgesehen. Deren Mittenfrequenzen f₁ bis fₙ entsprechen möglichst exakt den Modulationsfrequenzen f₁ bis fₙ. An den Ausgängen der Bandpaßfilter 8₁ bis 8ₙ stehen dann also elektrische Signale zur Verfügung, die die Intensität der von den Lichtquellen 1₁ bis 1ₙ stammenden Lichtanteile an dem empfangenen, durch das Objekt 6 transmittierten Anteil des diesem zugeführten Lichtes repräsentieren. Diese elektrischen Signale gelangen jeweils zu einer Signalaufbereitungsschaltung 9₁ bis 9ₙ, wo eine dem jeweiligen Untersuchungsfall angepaßte Signalaufbereitung erfolgt. Die Ausgangssignale der Signalaufbereitungsschaltungen 9₁ bis 9ₙ sind einem n:1-Analog-Multiplexer 29 zugeführt, dessen Ausgang mit dem Analog/Digital-Wandler 11 verbunden ist.

Der elektronischen Recheneinrichtung 12 steht also pro Abtastposition nicht ein den beiden Durchstrahlungsrichtungen entsprechendes Datenpaar, sondern eine der Anzahl n der unterschiedlichen Wellenlängen entsprechende Anzahl von Datenpaaren zur Verfügung, von denen jedes für eine andere Wellenlänge die beiden Durchstrahlungsrichtungen repräsentiert. Mit den aufgrund dieser Daten von der elektronischen Recheneinrichtung 12 erstellten und nach Bedarf auf dem Monitor 14 angezeigten Bildern kann in der zuvor beschriebenen Weise verfahren werden. Dabei beschränkt sich die Zusammenfassung von aus unterschiedlichen Bildern stammende Bildinformation zu einem neuen, kontrastreicheren Bild nicht auf die Bilder eines zu einer der Wellenlängen λ₁ bis λₙ gehörigen Bildpaares. Vielmehr besteht auch die Möglichkeit, Bildinformation aus zu unterschiedlichen Wellenlängen λ₁ bis λₙ gehörigen Bildern zu einem neuen, kontrastreicheren Bild zusammenzusetzen.

Während im Falle der Ausführungsbeispiele gemäß den Fig. 1 und 6 die Amplitudenmodulation des verwendeten Lichtes auch dazu dient, die Durchstrahlungsrichtung bzw. die jeweilige Wellenlänge λ₁ bis λₙ erkennen zu können, ist im Falle der übrigen Ausführungsbeispiele die Amplitudenmodulation des Lichtes nur vorgesehen, um Umgebungslichteinflüsse auszuschließen.

Im Falle der Ausführungsbeispiele gemäß den Fig. 1 bis 3 kann übrigens vorgesehen sein, daß die Recheneinrichtung 12 den Versatz der beiden Durchstrahlungsrichtungen kompensiert. Dies ist sozusagen durch eine Koordinatentransformation insbesondere dann sehr leicht möglich, wenn der Versatz nach Betrag und Richtung dem Abstand zwischen zwei benachbarten Abtastpositionen oder einem ganzzahligen Vielfachen davon entspricht.

Die im Zusammenhang mit den Ausführungsbeispielen ausschließlich beschriebene Amplitudenmodulation ist nicht das einzige brauchbare Modulationsverfahren, jedoch gestalten sich Modulation und Demodulation im Falle der Amplitudenmodulation besonders einfach.

Im Falle der beschriebenen Ausführungsbeispiele wird dem Photomultiplier 7 bzw. den Photomultiplieren 7₁ bis 7₂ das aus dem Objekt 6 austretende Licht jeweils über Lichtwellenleiter zugeführt. Es besteht aber auch die Möglichkeit, in nicht dargestellter Weise den Photomultiplier 7 bzw. die Photomultiplier 7₁ und 7₂ so anzuordnen, daß das aus dem Objekt 6 austretende Licht direkt aufgenommen werden kann. In diesem Falle müßten der Photomultiplier 7 bzw. die Photomultiplier 7₁ und 7₂ in geeigneter Weise an dem Träger 17 der Abtastmittel angebracht werden. Die Mittel zum Detektieren können anstelle eines Photomultipliers auch eine Photodiode oder ein CCD aufweisen.

Statt des Monitors 14 kann als Anzeigemittel ein LED-, LCD- oder Plasma-Display vorgesehen sein.

Die im Zusammenhang mit den Ausführungsbeispielen beschriebenen Mittel zum Einstrahlen von Licht in das Objekt, die eine oder mehrere Lichtquellen, einen oder mehrere Signalgeneratoren, verschiedene Lichtwellenleiter, eventuell die Umschalter 22 und 23 sowie eventuell den Lichtwellenleiter-Fan-In-Koppler 4 enthalten, können andersartig ausgebildet sein. So kann beispielsweise die Einstrahlung des Lichtes auch mittels einer Spiegelanordnung bewerkstelligt werden.

Auch die Ausbildung der Abtastmittel ist nur beispielhaft zu verstehen. Ein anderer Aufbau der Abtastmittel sowie von der beschriebenen Abtastbewegung abweichende Abtastbewegungen sind möglich.

Um zu vermeiden, daß der bzw. die Photomultiplier durch eine direkte Bestrahlung mit dem an sich in das Objekt einzustrahlenden Licht überbelichtet werden, besteht die Möglichkeit, unter Zuhilfenahme einer geeigneten Sensoreinrichtung die Abtastbewegung so zu steuern, daß von den an sich möglichen Abtastpositionen nur solche Abtastpositionen angefahren werden, bei denen sichergestellt ist, daß sich das Objekt 6 zwischen Lichtaustritts- und Lichteintrittszone befindet.

## Patentansprüche

1. Vorrichtung zur Untersuchung von Gewebe mit Licht, aufweisend
a) Mittel (1₁, 1₂, 3₁, 3₂; 1, 3₁, 3₂, 22, 24; 1, 22, 24, 26₁, 27₁; 1₁ bis 1ₙ, 4, 22, 24, 26₁, 27₁, 28₁ bis 28ₙ) zum bidirektionalen Durchstrahlen zu untersuchenden Gewebes mit Licht, und
b) Mittel (5₁, 5₂, 7₁, 7₂, 8₁, 8₂; 5₁, 5₂, 7, 8, 23, 25; 5₁, 5₂, 7₁, 7₂, 8₁, 82; 7, 8, 23, 25, 26₂ bis 26ₚ, 27₂ bis 27ₚ; 7, 8₁ bis 8ₙ, 23, 25, 26₂ bis 26ₚ, 27₂ bis 27ₚ) zum Detektieren , die dazu vorgesehen sind, Anteile des von den Mitteln (1₁, 1₂, 3₁, 3₂; 1, 3₁, 3₂, 22, 24; 1, 22, 24, 26₁, 27₁; 1₁ bis 1ₙ, 4, 22, 24, 26₁, 27₁, 28₁ bis 28ₙ) zum bidirektionalen Durchstrahlen ausgehenden Lichtes für beide Durchstrahlungsrichtungen zu detektieren.

2. Vorrichtung nach Anspruch 1 , bei der die bidirektionale Durchstrahlung wenigstens im wesentlichen quasi-gleichzeitig erfolgt.

3. Vorrichtung nach Anspruch 2 , deren Mittel (1₁, 1₂, 3₁, 3₂) zum Durchstrahlen in beiden Durchstrahlungsrichtungen gleichzeitig durchstrahlen.

4. Vorrichtung nach Anspruch 3 , bei der das Licht für die beiden Durchstrahlungsrichtungen jeweils unterschiedlich moduliert ist.

5. Vorrichtung nach Anspruch 4 , wobei das Licht für die beiden Durchstrahlungsrichtungen mit unterschiedlichen Modulationsfrequenzen (f₁, f₂) amplitudenmoduliert.

6. Vorrichtung nach einem der Ansprüche 1 bis 5, welche Blendenmittel (20, 21) aufweist, die alternierend für die eine oder die andere Durchstrahlungsrichtung das von den Mitteln (1₁, 1₂, 3₁, 3₂) zum Durchstrahlen ausgehende Licht von den Mitteln (5₁, 5₂, 7₁, 7₂, 8₁, 8₂) zum Detektieren fernhalten.

7. Vorrichtung nach einem der Ansprüche 1 bis 6, deren Mittel (1₁, 1₂, 3₁, 3₂) zum Durchstrahlen eine erste und eine zweite Lichtquelle (1₁, 1₂) und deren Mittel (5₁, 5₂, 7₁, 7₂, 8₁, 8₂) zum Detektieren eine erste und eine zweite Detektoreinrichtung (7₁, 7₂) aufweisen, wobei von der ersten Lichtquelle (1₁) das Licht in der einen Durchstrahlungsrichtung ausgeht und die erste Detektoreinrichtung (7₁) zur Detektion von Anteilen des von der ersten Lichtquelle (1₁) ausgehenden Lichtes vorgesehen ist, und wobei von der zweiten Lichtquelle (1₂) das Licht in der anderen Durchstrahlungsrichtung ausgeht und die zweite Detektoreinrichtung (7₂) zur Detektion von Anteilen des von der zweiten Lichtquelle (1₂) ausgehenden Lichtes vorgesehen ist.

8. Vorrichtung nach Anspruch 7, bei der die erste Lichtquelle (1₁) und die erste Detektoreinrichtung (7₁) einerseits und die zweite Lichtquelle (1₂) und die zweite Detektoreinrichtung (7₂) andererseits gleichzeitig aktiv sind.

9. Vorrichtung nach Anspruch 7, bei der die erste Lichtquelle (1₁) und die erste Detektoreinrichtung (7₁) einerseits oder die zweite Lichtquelle (1₂) und die zweite Detektoreinrichtung (7₂) andererseits alternierend aktiv sind.

10. Vorrichtung nach einem der Ansprüche 1, 2, 4 oder 5, deren Mittel (1, 3₁, 3₂, 22, 24; 1, 22, 24, 26₁, 27₁) zum Durchstrahlen eine Lichtquelle (1) aufweisen, der Lichtleitmittel (3₁, 3₂, 22, 24; 22, 24, 26₁, 27₁) zugeordnet sind, die das Licht der Lichtquelle (1) alternierend in der einen oder der anderen Durchstrahlungsrichtung aussenden.

11. Vorrichtung nach einem der Ansprüche 1 bis 5 oder 10, deren Mittel (5₁, 5₂, 7, 8, 23, 25; 7, 8, 23, 25, 26₂ bis 26ₚ, 27₂ bis 27ₚ; 7, 8₁ bis 8ₙ, 23, 25, 26₂ bis 26ₚ, 27₂ bis 27ₚ) zum Detektieren eine Detektoreinrichtung (7) aufweisen, der Lichtleitmittel (5₁, 5₂, 23, 25; 23, 25, 26₂ bis 26ₚ, 27₂ bis 27ₚ) zugeordnet sind, die dazu vorgesehen sind der Detektoreinrichtung (7) alternierend bezüglich der eine oder der anderen Durchstrahlungsrichtung zu detektierendes Licht zuzuleiten.

12. Vorrichtung nach Anspruch 10 oder 11, deren Lichtleitmittel (5₁, 5₂, 23, 25; 23, 25, 26₂ bis 26ₚ, 27₂ bis 27ₚ) faseroptische Mittel (3₁, 3₂, 5₁, 5₂, 24, 25, 26, 27) enthalten.

13. Vorrichtung nach Anspruch 12, deren faseroptischen Mittel (3₁, 3₂, 5₁, 5₂, 24, 25, 26, 27) ein Lichtleitfaserbündel (26, 27) enthalten, das wenigstens je eine von den Mitteln zum Durchstrahlen (1, 22, 24, 26₁, 27₁; 1₁ bis 1ₙ, 4, 22, 24, 26₁, 27₁, 28₁ bis 28ₙ) zum bidirektionalen Durchstrahlen ausgehendes Licht einer Durchstrahlungsrichtung und eine Anteile Lichtes der jeweils anderen Durchstrahlungsrichtung zu den Mitteln zum Detektieren leitende Lichtleitfaser (26₁ und 27₁ bzw. 26₂ bis 26ₚ und 27₂ bis 27ₚ) enthält.

14. Vorrichtung nach einem der Ansprüche 1 bis 13, die Abtastmittel (17, 18) aufweist, mittels derer die bidirektionale Durchstrahlung in einer Vielzahl von Abtastpositionen erfolgt.

15. Vorrichtung nach einem der Ansprüche 1 bis 14, die Auswertemittel (12) aufweist, denen die Ausgangssignale der Mittel (5₁, 5₂, 7₁, 7₂, 8₁, 8₂; 5₁, 5₂, 7, 8, 23, 25; 5₁, 5₂, 7₁, 7₂, 8₁, 8₂; 7, 8, 23, 25, 26₂ bis 26ₚ, 27₂ bis 27ₚ; 7, 8₁ bis 8ₙ, 23, 25, 26₂ bis 26ₚ, 27₂ bis 27ₚ) zum Detektieren zugeführt sind.

16. Vorrichtung nach Anspruch 15, bei der für die beiden Durchstrahlungsrichtungen ein Versatz der optischen Achsen vorliegt und deren Auswertemittel (12) den Versatz eliminieren.

17. Vorrichtung nach Anspruch 16, bei der der Versatz nach Betrag und Richtung gleich dem Versatz zwischen zwei benachbarten Abtastpositionen oder einem ganzzahligen Vielfachen davon ist.

18. Vorrichtung nach einem der Ansprüche 1 bis 17, deren Mittel (1₁ bis 1ₙ 4, 22, 24, 26₁, 27₁, 28₁ bis 28ₙ) zum bidirektionalen Durchstrahlen gleichzeitig Licht definierter unterschiedlicher Wellenlängen abgeben, wobei Signalverarbeitungsmittel vorgesehen sind, die anhand des Ausgangssignales der Mittel (7, 8₁ bis 8ₙ, 23, 25, 26₂ bis 26ₚ, 27₂ bis 27ₚ) zum Detektieren eine der Anzahl der unterschiedlichen Wellenlängen entsprechende Anzahl von Signalen bilden, von denen jedes dem Anteil des Lichtes einer der unterschiedlichen Wellenlängen an dem detektierten Anteil des von den Mitteln(1₁ bis 1ₙ, 4, 22, 24, 26₁, 27₁, 28₁ bis 28ₙ) zum Durchstrahlen Lichtes entspricht.

## Claims

1. Device for examining tissue with light, having
a) means (1₁, 1₂, 3₁, 3₂; 1, 3₁, 3₂, 22, 24; 1, 22, 24, 26₁, 27₁; 1₁ to 1ₙ, 4, 22, 24, 26₁, 27₁, 28₁ to 28ₙ) for bidirectionally irradiating with light tissue which is to be examined, and
b) means (5₁, 5₂, 7₁, 7₂, 8₁, 8₂; 5₁, 5₂, 7, 8, 23, 25; 5₁, 5₂, 7₁, 7₂, 8₁, 8₂; 7, 8, 23, 25, 26₂ to 26ₚ, 27₂ to 27ₚ; 7, 8₁ to 8ₙ, 23, 25, 26₂ to 26ₚ, 27₂ to 27ₚ) for detecting, which are provided in order to detect, for both directions of irradiation, portions of the light issuing from the means (1₁, 1₂, 3₁, 3₂; 1, 3₁, 3₂, 22, 24; 1, 22, 24, 26₁, 27₁; 1₁ to 1ₙ, 4, 22, 24, 26₁, 27₁, 28₁ to 28ₙ) for bidirectional irradiation.

2. Device according to claim 1, in which the bidirectional irradiation takes place at least substantially quasi-simultaneously.

3. Device according to claim 2, whose means (1₁, 1₂, 3₁, 3₂) for irradiating irradiate simultaneously in both directions of irradiation.

4. Device according to claim 3, in which the light for the two directions of irradiation is modulated differently in each case.

5. Device according to claim 4, with the light for the two directions of irradiation being amplitude-modulated with different modulation frequencies (f₁, f₂).

6. Device according to one of the claims 1 to 5, which has diaphragm means (20, 21) which, alternately for the one direction of irradiation or the other direction of irradiation keep away from the means (5₁, 5₂, 7₁, 7₂, 8₁, 8₂) for detecting the light issuing from the means (1₁, 1₂, 3₁, 3₂) for irradiating.

7. Device according to one of the claims 1 to 6, whose means (1₁, 1₂, 3₁, 3₂) for irradiating have a first light source (1₁) and a second light source (1₂) and whose means (5₁, 5₂, 7₁, 7₂, 8₁, 8₂) for detecting have a first detector device (7₁) and a second detector device (7₂), with the light issuing from the first light source (1₁) in the one direction of irradiation and the first detector device (7₁) being provided for detecting portions of the light issuing from the first light source (1₁), and with the light issuing from the second light source (1₂) in the other direction of irradiation and the second detector device (7₂) being provided for detecting portions of the light issuing from the second light source (1₂).

8. Device according to claim 7, in which the first light source (1₁) and the first detector device (7₁) on the one hand and the second light source (1₂) and the second detector device (7₂) on the other hand are simultaneously active.

9. Device according to claim 7, in which the first light source (1₁) and the first detector device (7₁) on the one hand or the second light source (1₂) and the second detector device (7₂) on the other hand are alternately active.

10. Device according to one of the claims 1, 2, 4 or 5, whose means (1, 3₁, 3₂, 22, 24; 1, 22, 24, 26₁, 27₁) for irradiating have one light source (1) to which are allocated optical waveguide means (3₁, 3₂, 22, 24; 22, 24, 26₁, 27₁), which emit the light of the light source (1) alternately in the one direction of irradiation or the other direction of irradiation.

11. Device according to one of the claims 1 to 5 or 10, whose means (5₁, 5₂, 7, 8, 23, 25; 7, 8, 23, 25, 26₂ to 26ₚ, 27₂ to 27ₚ; 7, 8₁ to 8ₙ, 23, 25, 26₂ to 26ₚ, 27₂ to 27ₚ) for detecting have one detector device (7) to which are allocated optical waveguide means (5₁, 5₂, 23, 25; 23, 25, 26₂ to 26ₚ, 27₂ to 27ₚ), which are provided in order to supply light to be detected to the detector device (7), alternately with regard to the one direction of irradiation or the other direction of irradiation.

12. Device according to claim 10 or 11, whose optical waveguide means (5₁, 5₂, 23, 25; 23, 25, 26₂ to 26ₚ, 27₂ to 27ₚ) contain fibre-optic means (3₁, 3₂, 5₁, 5₂, 24, 25, 26, 27).

13. Device according to claim 12, whose fibre-optic means (3₁, 3₂, 5₁, 5₂, 24, 25, 26, 27) contain an optical-fibre bundle (26, 27), which contains in each case at least one of the means for irradiating (1, 22, 24, 26₁, 27₁; 1₁ to 1ₙ, 4, 22, 24, 26₁, 27₁, 28₁ to 28ₙ) for bidirectionally irradiating light issuing in one direction of irradiation, and an optical fibre (26₁ and 27₁ or 26₂ to 26ₚ and 27₂ to 27ₚ) which guides portions of light of the respective other direction of irradiation to the means for detecting.

14. Device according to one of the claims 1 to 13, which has scanning means (17, 18), by means of which the bidirectional irradiation takes place in a plurality of scanning positions.

15. Device according to one of the claims 1 to 14, which has evaluating means (12), to which are supplied the output signals of the means (5₁, 5₂, 7₁, 7₂, 8₁, 8₂; 5₁, 5₂, 7, 8, 23, 25; 5₁, 5₂, 7₁, 7₂, 8₁, 8₂; 7, 8, 23, 25, 26₂ to 26ₚ, 27₂ to 27ₚ; 7, 8₁ to 8ₙ, 23, 25, 26₂ to 26ₚ, 27₂ to 27ₚ) for detecting.

16. Device according to claim 15, in which for the two directions of irradiation, the optical axes are offset with respect to each other and the evaluating means (12) of said device eliminate the offset.

17. Device according to claim 16, in which the offset is equal in terms of amount and direction to the offset between two adjacent scanning positions or an integral multiple thereof.

18. Device according to one of the claims 1 to 17, whose means (1₁ to 1ₙ, 4, 22, 24, 26₁, 27₁, 28₁ to 28ₙ) for bidirectionally irradiating simultaneously emit light of defined different wavelengths, with there being provided signal processing means which, with the aid of the output signal of the means (7, 8₁ to 8ₙ, 23, 25, 26₂ to 26ₚ, 27₂ to 27ₚ) for detecting, form a number of signals that corresponds to the number of different wavelengths, each of which signals corresponds to the portion of the light of one of the different wavelengths at the detected portion of the light from the means (1₁ to 1ₙ, 4, 22, 24, 26₁, 27₁, 28₁ to 28ₙ) for irradiating.

## Revendications

1. Dispositif pour examiner un tissu par de la lumière, comportant
a) des moyens (11, 12, 31, 32 ; 1, 31, 32, 22, 24 ; 1, 22, 24, 261, 271 ; 11 à 1n, 4, 22, 24, 261, 271, 281 à 28n) pour exposer de manière bidirectionnelle le tissu à examiner à de la lumière, et
b) des moyens (51, 52, 71, 72, 81, 82 ; 51, 52, 7, 8, 23, 25 ; 51, 52, 71, 72, 81, 82 ; 7, 8, 23, 25, 262 à 26p, 272 à 27p ; 7, 81 à 8n, 23, 25, 262 à 26p, 272 à 27p) de détection, qui sont prévu pour détecter des composantes de la lumière issues des moyens (11, 12, 31, 32 ; 1, 31, 32, 22, 24 ; 1, 22, 24, 261, 271 ; 11 à 1n, 4, 22, 24, 261, 271, 281 à 28n) d'exposition bidirectionnelle, pour les deux directions d'exposition.

2. Dispositif suivant la revendication 1, dans lequel l' exposition bidirectionnelle s'effectue au moins sensiblement quasiment simultanément.

3. Dispositif suivant la revendication 2, dont les moyens (11, 12, 31, 32) d' exposition dans les deux directions d' exposition envoient de la lumière en même temps.

4. Dispositif suivant la revendication 3, dans lequel la lumière pour les deux directions d' exposition est modulée de manière différente.

5. Dispositif suivant la revendication 4, dans lequel la lumière pour les deux directions d' exposition est modulée en amplitude à des fréquences (f1, f2) de modulation différentes.

6. Dispositif suivant l'une des revendications 1 à 5, qui comporte des moyens (20, 21) d'écran, qui éloignent en alternance pour l'une ou l'autre direction d' exposition la lumière sortant des moyens (11, 12, 31, 32) d'exposition des moyens (51, 52, 71, 72, 81, 82) de détection.

7. Dispositif suivant l'une des revendications 1 à 6, dont les moyens (11, 12, 31, 32) d' exposition comportent une première et une seconde source de lumière (11, 12) et dont les moyens (51, 52, 71, 72, 81, 82) de détection comportent un premier et un second dispositif (71, 72) de détection, la lumière sortant de la première source de lumière (11) dans l'une des directions d'exposition et le premier dispositif (71) de détection étant prévus pour la détection de composantes de la lumière sortant de la première source (11) de lumière, et la lumière sortant de la seconde source (12) de lumière dans l'autre direction d'exposition et le second dispositif (72) de détection étant prévus pour la détection de composantes de la lumière sortant de la seconde source (12) de lumière.

8. Dispositif suivant la revendication 7, dans lequel la première source (11) de lumière et le premier dispositif (71) de détection d'une part et la seconde source (12) de lumière et le second dispositif (72) de détection d'autre part sont actifs en même temps.

9. Dispositif suivant la revendication 7, dans lequel la première source (11) de lumière et le premier dispositif (71) de détection d'une part et la seconde source (12) de lumière et le second dispositif (72) de détection d'autre part sont actifs en alternance.

10. Dispositif suivant l'une des revendications 1, 2, 4 ou 5, dont les moyens (1, 31, 32, 22, 24 ; 1, 22, 24, 261, 271) d' exposition comportent une source (1) de lumière, à laquelle sont associés des moyens (31, 32, 22, 24 ; 22, 24, 261, 271) de guidage de la lumière, qui émettent la lumière de la source (1) de lumière en alternance dans l'une ou l'autre directions d'exposition.

11. Dispositif suivant l'une des revendications 1 à 5 ou 10, dont les moyens (51, 52, 7, 8, 23, 25 ; 7, 8, 23, 25, 262 à 26p, 272 à 27p ; 7, 81 à 8n, 23, 25, 262 à 26p, 272 à 27p) de détection comportent un dispositif (7) de détection, auquel sont associés des moyens de guidage de la lumière (51, 52, 23, 25 ; 23, 25, 262 à 26p, 272 à 27p) qui sont prévus pour envoyer la lumière à détecter au dispositif (7) de détection en alternance par rapport à l'une ou l'autre direction d'exposition.

12. Dispositif suivant la revendication 10 ou 11, dont les moyens de guidage de la lumière (51, 52, 23, 25 ; 23, 25, 262 à 26p, 272 à 27p) comprennent des moyens de fibres optiques (31, 32, 51, 52, 24, 25, 26, 27).

13. Dispositif suivant la revendication 12, dont les moyens de fibres optiques (31, 32, 51, 52, 24, 25, 26, 27) comprennent un faisceau (26, 27) de fibres optiques, qui comprend au moins une fibre optique (261 et 271 ou 262 à 26p et 272 à 27p) guidant de la lumière, sortant des moyens d'exposition (1, 22, 24, 261, 271 ; 11 à 1n, 4, 22, 24, 261, 271, 281 à 28n) pour l'exposition bidirectionnelle, d'une direction d'exposition et une fibre optique guidant une composante de la lumière de l'autre direction d'exposition vers les moyens de détection.

14. Dispositif suivant l'une des revendications 1 à 13, qui comporte des moyens de balayage(17, 18), au moyen desquels l'exposition bidirectionnelle s'effectue dans une multiplicité de positions de balayage.

15. Dispositif suivant l'une des revendications 1 à 14, qui comporte des moyens (12) d'exploitation auxquels sont envoyés les signaux de sortie des moyens (51, 52, 71, 72, 81, 82 ; 51, 52, 7, 8, 23, 25 ; 51, 52, 71, 72, 81, 82 ; 7, 8, 23, 25, 262 à 26p, 272 à 27p ; 7, 81 à 8n, 23, 25, 262 à 26p, 272 à 27p) de détection.

16. Dispositif suivant la revendication 15, dans lequel il existe un décalage des axes optiques pour les deux directions d'exposition et leurs moyens (12) d'exploitation éliminent le décalage.

17. Dispositif suivant la revendication 16, dans lequel le décalage est égal, en module et en direction au décalage entre deux positions voisines de balayage ou est égal à un multiple entier de celui-ci.

18. Dispositif suivant l'une des revendications 1 à 17, dont les moyens (11 à 1n, 4, 22, 24, 261, 271, 281 à 28n) d'exposition bidirectionnelle émettent simultanément de la lumière de longueur d'onde différente définie, des moyens de traitement des signaux étant prévus, lesquels forment, sur la base du signal de sortie des moyens (7, 81 à 8n, 23, 25, 262 à 26p, 272 à 27p) de détection un nombre de signaux correspondant au nombre des longueurs d'ondes différentes, dont chacun correspond à la composante de lumière d'une des longueurs d'ondes différentes sur la composante détectée de la lumière envoyée par les moyens (11 à 1n, 4, 22, 24, 261, 271, 281 à 28n) d'exposition.
